(19) Europäisches Patentamt
European Patent Office
Office européen des brevets

(11) EP 4 578 917 B1

(12) **EUROPEAN PATENT SPECIFICATION**

(45) Date of publication and mention of the grant of the patent:
**22.04.2026 Bulletin 2026/17**

(21) Application number: **24222197.6**

(22) Date of filing: **20.12.2024**

(51) International Patent Classification (IPC):
***C09B 61/00*** (2006.01) ***C09D 11/037*** (2014.01)
***C12N 1/20*** (2026.01) ***D06P 1/34*** (2006.01)
***D06P 5/00*** (2006.01)

(52) Cooperative Patent Classification (CPC):
**C12N 1/205; C09B 61/00; C09B 67/0066; C09D 11/037; C12N 1/20; D06P 1/34; D06P 5/001;**
C12R 2001/01

(54) **MICROBIAL INK, PRODUCTION AND APPLICATION THEREOF**

MIKROBIELLE TINTE, HERSTELLUNG UND VERWENDUNG DAVON

ENCRE MICROBIENNE, SA PRODUCTION ET SON APPLICATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **22.12.2023 AT 510542023**

(43) Date of publication of application:
**02.07.2025 Bulletin 2025/27**

(83) **Declaration under Rule 32(1) EPC (expert solution)**

(73) Proprietor: **VTL GmbH**
**1030 Wien (AT)**

(72) Inventors:
- **FLECK, Karin**
**1030 Wien (AT)**
- **RAUSCHER, Mascha**
**1140 Wien (AT)**
- **SCHOPF, Erich**
**1220 Wien (AT)**
- **SMIRNOVA, Uliana**
**1170 Wien (AT)**
- **GYÖRKE, Gábor**
**4020 Linz (AT)**
- **GRANATIER, Marianna**
**1220 Wien (AT)**
- **LUCHTMAN, Laura**
**3037 SH Rotterdam (NL)**
- **SIEBENHAAR, Ilfa**
**1055 HP Amsterdam (NL)**

(74) Representative: **Clark, Claudia et al**
**REDL Life Science Patents OG**
**Donau-City-Straße 11**
**1220 Wien (AT)**

(56) References cited:
**WO-A1-2004/048589 FR-A1- 3 062 130**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE INVENTION

The invention relates to the field of inks. More specifically, the invention relates to an ink comprising microbial biomass, a production method of such an ink, and applications of such an ink.

### BACKGROUND OF THE INVENTION

The textile industry is one of the largest global industrial polluters and has one of the largest water footprints. The dyeing process is one of the main culprits of polluting rivers and lakes, posing a hazard for textile workers and eventually for the end consumers. Estimations reveal the use of 79 billion cubic meters of water within the global textile and clothing industry in 2015 corresponding to one third of EU's whole economy need in 2017.

Since prehistoric times, color plays an extraordinary and important role, evidently preserved in archaeological discoveries e.g., the Paleolithic cave paintings of Altamira. Our ancestors used natural such as mineral pigments to color virtually their environment - sculptures, pottery, clothes, and textiles - and even themselves, their skin. First evidence for printing processes - also on textiles - with ink is suggested to date back to ancient China approx. 250 BC after the development of handwriting, but only reached the European continent in the Middle Ages. "The Gutenberg Bible" is a famous example of the printing evolution in Europe, highly outstanding for its accuracy and quality.

Potential impact on human health, environment, and sustainability in general are vital concerns. Consumers become more and more aware of product lifecycles and harmful effects caused by chemicals. Within the screen-printing industry, inks based on polyvinyl chloride (PVC, plastisol inks) are commonly used but production results in highly toxic and carcinogenic compounds, e.g., dioxin. Phthalate, a softening component of plastisol inks, is released into the environment during printing and curing with damaging effects. Carbon-black, one of the most widely used chemical printing ink, is a potential carcinogen. Solvent-based printing inks release volatile organic compounds (VOCs) during the printing process and contribute to air pollution.

EP0252002A2 discloses a method for dyeing a substrate comprising applying to the substrate a biomass containing indigo in which the dye has been produced by a microorganism without isolating the dye from the biomass before dying.

FR3062130A1 discloses the use of actinorhodin and/or the derivatives thereof as a colouring agent.

WO2004048589A1 discloses the isolation of compounds such as carotenoids and flavonoids from a marine bacterium belonging to the genus *Pseudoalteromonas* sp.

However, there is no sustainable method known for producing an ink.

Thus, there is an urgent need for new biological and sustainable production systems for inks and for biological and sustainable printing on substrates such as textiles.

### SUMMARY OF THE INVENTION

It is the objective of the present invention to provide a biological system for the sustainable production of inks and for a sustainable colorization or printing method of substrates.

The objective is solved by subject matter of the present invention.

It has been surprisingly shown that pigment producing microorganisms can be used directly in ink formulations. Specifically, the inventors of the present invention found that pigment producing microorganisms can be mixed with binders leading to a homogenous paste which can be used as ink for printing purposes. The ink comprising biomass as provided herein did surprisingly not clog meshes used commonly for screen printing purposes and did not dry too quickly although microbial biomass is present in the ink.

According to the invention there is provided a method of producing an ink, said method comprising the steps of:

i. cultivating a pigment producing microorganism in a cultivation medium for producing biomass of said microorganism comprising the pigment;
ii. separating the produced biomass from the cultivation medium;
iii. inactivating the microorganism of the biomass;
iv. drying the biomass;
v. mixing the dried powder with a binder;

wherein the pigment is membrane-bound.

According to a specific aspect, said microorganism is a bacterium, specifically a bacterium belonging to the phylum *Actinomycetota,* more specifically to the class *Actinomycetia,* even more specifically the bacterium is Kocuria sp.,

preferably *Kocuria marina* and even more preferably *Kocuria marina* O13.

According to a preferred aspect, the pigment producing microorganism is an isolated bacterium belonging to the species *Kocuria marina* deposited under the number CBS 150888 at the Westerdijk Fungal Biodiversity Institute on December 19, 2023.

Specifically, the pigment is anchored in a membrane of the microorganism described herein.

Specifically, in the method described herein, cultivating is performed at least until a color of a pigment is detected in the biomass.

According to an aspect of the method described herein, the cultivation medium is a liquid medium.

Specifically, the pigment produced by the microorganism is water-insoluble.

According to a further aspect, the method further comprises the step of producing a powder from the dried biomass before mixing the dried biomass with the binder.

Specifically, in the method described herein, separating the produced biomass from the cultivation medium is performed by one or more of filtration, centrifugation, or disk separation.

In a specific aspect, the binder comprises starch, gum arabic (GA), carboxymethylcellulose (CMC), guar gum (GG), chitosan, acetic acid, kaolin, methyl cellulose, sodium benzoate, or a combination thereof.

According to a preferred aspect, the binder comprises starch, specifically pure starch. Preferably, the binder is starch.

Preferably, in the method described herein a binder comprising starch is used. Non-limiting examples of commercially available binders comprising starch are Togis #8160 and #8161 (TOGIS thickener containing pure starch, from Livos, DE).

Specifically, the color of the biomass is preserved.

Further provided herein is an ink produced according to the method described herein.

Further provided herein is the use of an ink described herein for changing the color of a substrate.

In a further aspect, provided herein is a method of printing ink on a substrate, said method comprising the steps of:

i. providing an ink described herein;
ii. contacting the ink with the substrate; and
iii. fixing the ink on the substrate.

Specifically, contacting is performed by screen printing, block printing, or 3D-printing.

Specifically, fixing is performed by heat treatment of the substrate, specifically at a temperature in the range of 60°C to 121°C.

According to a further aspect, the herein described methods are used for a substrate, wherein the substrate is a textile material.

Further provided herein is an ink comprising a biomass of a pigment producing microorganism, and a binder, such as for example starch, gum arabic (GA), carboxymethylcellulose (CMC), guar gum (GG), chitosan, acetic acid, kaolin, methyl cellulose, sodium benzoate, or a combination thereof, wherein the pigment is membrane-bound. Preferably, the binder comprises or consists of starch. According to a specific aspect, said microorganism is a bacterium, specifically a bacterium belonging to the phylum *Actinomycetota,* more specifically to the class *Actinomycetia,* even more specifically the bacterium is Kocuria sp., preferably *Kocuria marina.*

In a yet further aspect, for the ink described herein, the pigment producing microorganism is an isolated bacterium belonging to the species *Kocuria marina* deposited under the number CBS 150888 at the Westerdijk Fungal Biodiversity Institute on December 19, 2023 (*Kocuria marina O13*).

According to a further aspect, herein provided is also an isolated bacterium belonging to the species *Kocuria marina* deposited under the number CBS 150888 at the Westerdijk Fungal Biodiversity Institute on December 19, 2023.

<u>FIGURES</u>

**Figure 1:** overview of an exemplary screen-printing process. Screen printing describes the process of pressing whereby the colored ink, through a screen-printing paste that serves as a binder, is pressed through a mesh screen to create a printed pattern or design. A rubber blade, also known as squeegee, presses the ink paste through the mesh screen onto the fabric's surface imprinting the stencils design. The printed textile is dried, thereby curing ink and creating a smooth, colorfast finish. The final product is checked and washed thoroughly to remove any residue.

**Figure 2:** phase-contrast microscopy of *K. marina* O13 (deposited under the number CBS 150888 at the Westerdijk Fungal Biodiversity Institute on December 19, 2023) in TB medium after 16 hours at 28° C.

**Figure 3:** phase-contrast microscopy of *K. marina* O13 in LB medium after 16 hours at 28° C.

**Figure 4:** phase-contrast microscopy of *K. marina* O13 as concentrated harvest.

**Figure 5:** growth curve of *K. marina* O13 in LB medium during 20 hours batch cultivation at 28°C (left). In the course of the fermentation, the broth turns orange due to increased biomass which is visible in Fig. 5 (right) showing an orange-colored fermentation broth in the bioreactor.

**Figure 6:** result of the screen printing using C19 orange (ink comprising *Kocuria marina* O13). Thereby, an ink comprising 1.6 g / 8% pigment was used. As binders, 20 g of binder TPT or TGB was used. A light beige color formation is shown using binder TPT and a yellow to orange color formation using binder TGB. The following is shown as A, B, C, and D: A = n/a, control group; B = heat press; C = heat press + rinsing with water (after 24h); D = heat press + washing with mild laundry detergent (after 24h).

**Figure 7:** result of the screen printing using C18 purple (ink comprising *Chromobacterium vaccinii*). Thereby, an ink comprising 0.8 g / 4% pigment was used. As binder, 20 g of binder TPT was used. As textile samples 77% cotton/ 23% polyester and 78% recycled PA/22% elastane were used. Herein shown is a blue and not fully covering color formation using the textile sample 77% cotton/ 23% polyester. Using the textile sample 78% recycled PA/22% elastane a fully covering blue color formation is shown. The following is shown as A, B, C, and D: A = n/a, control group; B = heat press; C = heat press + rinsing with water (after 24h); D = heat press + washing with mild laundry detergent (after 24h).

**Figure 8:** pigment extracted from *Kocuria marina* O13. The figure shows an orange-colored liquid in a bottle.

**Figure 9:** UV-VIS spectrum of pigment extracted from *Kocuria marina* O13

**Figure 10:** UV-VIS spectrum of different fractions separated by column chromatography from the pigment extract of *Kocuria marina* O13.

## DETAILED DESCRIPTION

The invention is defined in the accompanying claims.

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017); Berg et al, "Stryer Biochemie" Springer Verlag, 2018; Itten J., 2021, "Kunst der Farbe"; St. Clair K., 2017, "Het geheime leven van kleuren"; Booth A., 2017, "The Wild Dyer"; Wada S., 2011, "Dictionary Of Color Combinations" vol. 1; Perryman L., 2021, "The Colour Bible".

The subject matter of the claims specifically refers to artificial products or methods employing or producing such artificial products, which may be variants of native (wild-type) products. Though there can be a certain degree of sequence identity to the native structure, it is well understood that the materials, methods, and uses of the invention, e.g., specifically referring to isolated nucleic acid sequences, amino acid sequences, expression constructs, transformed host cells and modified proteins and enzymes, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

According to one aspect, steps in methods described herein are sequential steps.

The term **"pigment"** as used herein refers to a chemically inert substance that is insoluble in water and can color a surface when mixed with the binder.

The term **"ink"** as used herein refers to a gel, composition, or solution that contains at least one colorant, such as a dye or pigment, and is used to color a surface to produce e.g., an image, text, or design.

According to one aspect, the herein described biomass comprises a pigment producing microorganism.

The term **"biomass"** as used herein refers to a mass of microorganisms. The term includes whole cells, partial cells, disrupted cells, or parts of cells.

According to one aspect, the herein described biomass comprises whole cells or partial cells. According to a specific aspect, partial cells may comprise up to 20, 30, 40, 50, 60, 70, or 90 % of a whole cell.

According to a specific aspect, the herein described ink may comprise whole cells or partial cells of the microorganism. Specifically, the partial cells may be cells having a disrupted cell membrane.

Specifically, the ink comprises microbial cells containing the pigment. The cells may be intact microbial cells or parts thereof containing the pigment. Specifically, the ink is bound by and/or within the microbial cells.

According to one aspect, the pigment producing microorganism may be a bacterium, or a fungus.

According to one aspect, the herein described pigment producing microorganism is a bacterium.

According to one aspect, the herein described pigment producing microorganism is a pigment producing bacterium.

According to one aspect, the herein described pigment producing bacterium belongs to the phylum *Actinomycetota.* Specifically, to the class of *Actinomycetia.*

According to a specific aspect, the pigment producing bacterium is *Kocuria* sp.

According to a specific aspect, the pigment producing bacterium is *Kocuria marina.*

According to a specific aspect, the pigment producing microorganism is an isolated bacterium belonging to the species *Kocuria marina* deposited under the number CBS 150888 at the Westerdijk Fungal Biodiversity Institute on December 19, 2023.

According to one aspect, described herein is an isolated bacterium, herein also referred to as *Kocuria marina* 013, belonging to the species *Kocuria marina* deposited under the number CBS 150888 at the Westerdijk Fungal Biodiversity Institute on December 19, 2023.

According to one aspect, the isolated bacterium belonging to the species *Kocuria marina* deposited under the number CBS 150888 at the Westerdijk Fungal Biodiversity Institute on December 19, 2023 described herein is a pigment producing bacterium.

According to one aspect, the herein described microorganism produces a pigment or a dye.

According to one aspect, the herein described pigment producing microorganism produces a membrane-anchored pigment. In other words, the pigment present in the biomass of the pigment producing microorganism is anchored in its membrane.

According to one aspect, the herein described pigment producing microorganism produces a water-insoluble pigment.

According to one aspect, the herein described pigment producing microorganism keeps the pigment in the biomass during cultivation in a liquid medium. In other words, the pigment stays in the biomass during cultivation and does not accumulate in the cultivation medium. As described herein, the pigment is membrane-bound. Specifically, the pigment is anchored in a membrane of the pigment producing microorganism. Specifically, there is no water-soluble part of the pigment expressed into the cultivation medium.

Specifically, *K. marina* O13 is a pigment producing microorganism. Specifically, *K. marina* O13 produces a membrane-bound pigment.

The term **"cultivating"** as used herein refers to the growth under conditions outside of the natural environment of the herein described microorganism.

According to one aspect, cultivating of the pigment producing microorganism is performed in a liquid medium.

In general, the skilled person is aware of suitable media for cultivating microorganism.

According to a specific aspect, the cultivation medium comprises a carbohydrate source.

According to a specific aspect, the cultivation medium comprises glucose as carbohydrate source.

According to a specific aspect, the cultivation medium may comprise a different carbohydrate source than glucose or the combination of glucose with a different carbohydrate source. Specifically, the cultivation medium may comprise maltose instead of glucose, or a combination of maltose and glucose.

According to a specific aspect, the cultivation medium comprises peptone.

According to a specific aspect, the cultivation medium comprises 1%, 5%, 10%, 15%, 20%, 1 to 5%, 1 to 10%, 5 to 10%, 10 to 15%, 5 to 15%, or 1 to 15% (m/v) peptone.

According to a specific aspect, the cultivation medium may comprise any other component commonly used in cultivation media. Non-limiting examples of such components are buffers, salts, yeast extract, malt extract, amino acids, starch, soybean meal, potato extract, rice extract, casein, dextrin, and antibacterial agents.

According to a specific aspect, cultivating is performed in a medium comprising 10 g $l^{-1}$ peptone of casein, 5 g $l^{-1}$ yeast extract, and 5 g $l^{-1}$ sodium chloride.

According to a specific aspect, cultivating is performed in a medium comprising 12 g/L tryptone, 24 g/L yeast extract, 9.4 g/L potassium phosphate (dibasic) and 2.2 g/L potassium phosphate (monobasic).

According to one aspect, cultivating is performed at a temperature in the range of 10°C to 35°C. Specifically, in the range of 15°C to 35°C. Specifically, in the range of 15°C to 30°C. Specifically, in the range of 15°C to 30°C. Specifically, in the range of 20°C to 30°C. Specifically, in the range of 25°C to 30°C. Specifically, in the range of 25°C to 28°C. Specifically, at 15, 16, 17, 18, 19, 20, 21, 22, 23, 24, 25, 26, 27, 28, 29, 30, 31, 32, 33, 34, or 35°C.

According to a specific aspect, the cultivation medium has a pH in the range of pH 5.0 to 8.5. Specifically, in the range of pH 6.0 to 8.0. Specifically, in the range of pH 6.5 to 7.5. Specifically, the pH is pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, or 8.5.

According to a specific aspect, the pH of the cultivation medium is maintained during cultivation. The pH can be maintained by any known method such as using a buffered cultivation medium and/or adding acids or bases. The pH may be controlled by a control unit of a bioreactor.

According to one aspect, cultivating is performed at least until a color of a pigment is detected in the biomass.

Such a detection of a pigment can be performed e.g., by visually inspecting color formation.

According to one aspect, inactivating may be performed by heating the biomass or the cultivation medium comprising the biomass to a temperature of about 121°C for at least 20 minutes.

According to one aspect, the microorganisms present in the produced biomass may be inactivated prior to separating the produced biomass from the cultivation medium or after separating the produced biomass from the cultivation medium.

According to one aspect, separating the produced biomass from the cultivation medium may be performed by any suitable method for separating biomass. Non-limiting examples of separation methods are filtration, disk separation, and centrifugation.

According to one aspect, the separated biomass may be washed e.g., with water or a saline solution. Such a saline solution may comprise 0.9% sodium chloride.

According to a specific aspect, the separated biomass may be washed twice or several times.

According to a specific aspect, the cells of the biomass are disrupted.

According to a specific aspect, the method described herein further comprises the disruption of the cells of the biomass.

According to a specific aspect, the method described herein further comprises a step of enriching the pigment comprising biomass. Specifically, an enrichment of part of the cells comprising the pigment.

According to a specific aspect, the method described herein further comprises a step of extracting pigment comprising cells parts.

According to a specific aspect, extracting pigment comprising cell parts may be performed by liquid extraction, supercritical fluid extraction, or absorption.

According to one aspect, drying the biomass may be performed by any suitable drying method. Specifically, drying is performed by lyophilization.

According to a specific aspect, the biomass is dried to a moisture content of 5% or lower.

According to one aspect, inactivating and drying may be performed in one single step. For example, drying the biomass may also lead to inactivating the biomass, or inactivating the biomass may also lead to drying of the biomass.

According to one aspect, producing a powder from the dried biomass may be performed by any suitable method for producing a powder. Specifically, a powder is produced by grinding with a blade grinder.

According to one aspect, the particles present in the powder have a particle size range of 10-300 μm.

According to one aspect, described herein is a method of producing an ink, wherein the color of the biomass is preserved. For example, if the biomass comprises an orange-colored pigment and thus, the biomass appears orange-colored, the ink produced with this biomass is an orange-colored ink.

According to one aspect, producing the ink described herein is performed in such a way that the biomass is not charred. In other words, in such a way that the color is preserved.

According to one aspect, inactivating the microorganism is performed in such a way that the biomass is not charred. In other words, in such a way that the color is preserved.

According to one aspect, drying the biomass is performed in such a way that the biomass is not charred. In other words, in such a way that the color is preserved.

According to one aspect, drying the biomass is performed by lyophilization.

In general, lyophilization of biomass may lead to dry biomass in powder-form.

According to one aspect, the dried biomass is mixed with a binder. Specifically, mixing is performed until homogenous distribution of dried biomass and binder.

According to a specific aspect, mixing may be performed by any suitable method known in the art.

According to one aspect, the dried biomass is mixed directly with the binder.

According to one aspect, the binder comprises water.

According to one aspect, the binder is water-based.

According to an alternative aspect, the dried biomass is suspended and mixed with water and thereafter, the binder is mixed with the dried biomass suspended in water.

According to an alternative aspect, the dried biomass is mixed with a binder and with water.

The term **"binder"** as used herein refers to any material or substance that holds other material together to form a cohesive whole. Specifically, the term "binder" as used herein in the context of dried biomass, or powder obtained from biomass refers to any material or substance that holds the herein described dried biomass, or powder obtained from the dried biomass together to form a cohesive whole. Binders can improve the attachment of pigment to the fabric. A binder may also be any material or substance that holds the herein described pigment, or dye together to form a cohesive whole. The term "binder" as used herein also includes thickeners.

According to one aspect, the binder comprises gum arabic (GA), carboxymethylcellulose (CMC), guar gum (GG), starch, chitosan, acetic acid, kaolin, methyl cellulose, sodium benzoate or a combination thereof.

The term **"gum arabic"** (abbreviated as "GA", also known as gum acacia, gum sudani, and Senegal gum) as

used herein refers to a natural gum harvested from trees. In general, gum arabic is a mixture of glycoproteins and polysaccharides, predominantly polymers of arabinose and galactose.

The term **"carboxymethylcellulose"** (abbreviated as "CMC", also known as cellulose gum) is a cellulose derivative with carboxymethyl groups (-CH2-COOH) bound to some of the hydroxyl groups of the glucopyranose monomers that make up the cellulose backbone. The term may also refer to a salt thereof such as to its sodium salt, sodium carboxymethyl cellulose.

The term **"guar gum"** (abbreviated as "GG", also known as guaran) is a galactomannan polysaccharide extracted from guar beans.

The term **"Chitosan"** refers to a natural polymer derived from the shells of crustaceans, used in various applications due to its biodegradability and non-toxicity. It has the structure

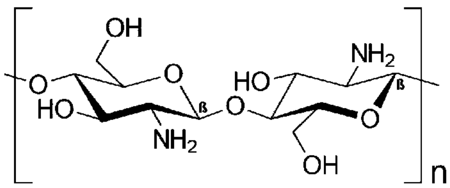

The term **"acetic acid"** is an organic compound with the formula $CH_3COOH$, commonly known as the main component of vinegar besides water.

The term **"kaolin"** refers to a clay mineral, part of the group of industrial minerals, used in a variety of products from paper to cosmetics.

The term **"methyl cellulose"** refers to a chemical compound derived from cellulose, used as a thickener and emulsifier in various food and cosmetic products. It has the structure

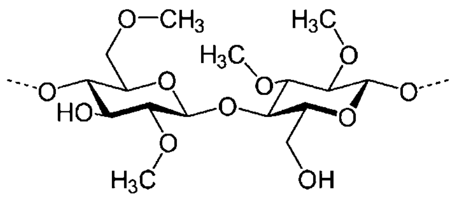

**Sodium benzoate** has the formula $C_6H_5COONa$.

According to one aspect, the concentration of dried biomass in the ink is in the range of 4 to 8 % (%w/w). According to a specific aspect, the concentration of the dried biomass in the in the range of 4 to 8 % (%w dried biomass/w binder).

According to an aspect, the present inks have a viscosity that allows them to pass through the mesh without bleeding. Viscosity is usually measured in centipoise (cP) and, as mentioned, is usually between 10,000 and 15,000 cP.

For textile printing in centimetres, the number of meshes usually ranges from about 43 to 63 threads per centimetre (TPcm) for most standard images. For printing finer details, around 90 TPcm may be used. The viscosity can be adjusted to be thinner for higher meshes and thicker for lower meshes. Specifically for tests, d an 'all-round' grid of 54 TPcm may be used to ensure that all inks, such as the biomass combined with the type of binder could pass through the meshes of the screen printing window.

Centipoise (cP) is a unit of dynamic viscosity, which measures the resistance of a fluid to flow or, in simpler terms, how "thick" or "thin" a fluid is. It is part of the centimeter-gram-second (CGS) system of units. 1 poise (P) is defined as the viscosity of a fluid in which a force of 1 dyne per square centimeter is needed to move a layer of the fluid along a parallel layer 1 centimeter away at a speed of 1 centimeter per second. It is equivalent to 1 $cm^{-1}\cdot g\cdot s^{-1}$. Since a poise is relatively large for everyday measurements, centipoise is more commonly used, where 1 poise = 100 centipoise. Water at room temperature has a viscosity of about 1 centipoise, which provides a reference point for understanding viscosity measurements. For example, fluids with a viscosity less than 1 cP flow more easily than water, and those with a viscosity higher than 1 cP flow less easily. In screen printing, knowing the viscosity of the ink is crucial because it affects how the ink will behave during the printing process, including how it transfers from the screen to the substrate

The addition of thickeners can increase viscosity, beneficial for coverage on dark fabrics or printing thicker layers.

According to the fabric types used for printing, ink absorption by different fabrics may differ, thus the ink can be adapted accordingly. E.g. cotton may require thicker inks than polyester.

The presence of more pigment may also increase the viscosity and opacity of the ink, sometimes requiring adjustments to the recipe for proper screen passage. Such adjustment can be performed according to known methods.

According to one aspect, described herein is the use of an ink as described herein for changing the color of a substrate.

The term **"changing the color"** as used herein refers to a change in the color of a substrate. Thus, the visual appearance of the substrate changes. Thereby, the color of the whole substrate or a specific part of the substrate may be obtained.

In general, colors can be described using color models. The CYMK model is one method to describe a color. The CYMK model reflects the colors cyan, yellow, magenta, and key, wherein key is black. An alternative method for describing a color the HSL color model. HSL stands for hue, saturation, and lightness.

The term **"color space"** as used herein refers to a measure to define a change is color or distance. A color space in which the perceptual difference between colors is directly related to distances between colors as represented by points in the color space, i.e. a uniform color space (Hans G. Völz (2001). Industrial Color Testing: Fundamentals and Techniques. Wiley-VCH. ISBN 3-527-30436-3) The term "ΔE" as used herein refers to color difference or color distance. The term "CIE color space" as used herein refers to a color space defined by CIE (Commission internationale de l'éclairage). Firstly, this was the CIELAB color space, but later formulas for the calculation of ΔE are using the CIELCH color space which is derived from the CIELAB color space. Both are uniform color spaces.

In the context of textiles, ΔE may be a critical measure of quality control. Textiles often require consistent color reproduction, which can be challenging given the variables in fabric production and the dyeing process. A low ΔE value is especially important in situations where color matching is required, such as in fashion, where different batches of fabric need to look the same, or in brand logos where the exact shade is part of the corporate identity. The CIE color space can be used in textile printing to provide a standard method for defining and measuring colors. This is important when colors need to be matched between different batches of textiles or when a design moves from design to production. Textiles do not always reflect colors in the same way as they appear in the CIE color space due to differences in material properties, so adjustments are often made to account for the specific behavior of textile dyes and the substrate. PMS and/or CYMK colors can be used as a reference in comparison to the new natural colors of microorganisms.

One example of changing the color of a specific part of a substrate is printing an ink on a substrate.

In general, in screen printing, a screen frame or window is used. For each color, the print is applied to the textile via the screen frame. The number of frames depends on the number of colors in the print file. Commonly, print is used in PMS (1, 2, 3 or 4 colors) and vector graphics are used. The colors are built up per layer (screen window). Because the colors are built up on top of each other, the colors blend together on the textile. Thereby, the term "PMS" refers to the Pantone Matching System which is a universal color-coding system and commonly used in the field. Pantone colors are standardized colors where each color has a unique code, making it easy to communicate and reproduce specific shades.

The term **"printing"** as used herein refers to the application of a dye, pigment, or ink to a distinct and selected area of a substrate. Specifically, textile printing refers to the application of a dye, pigment, or ink to a distinct and selected area of a textile fabric and results in a desired pattern instead of uniformly dyeing the whole fabric.

In general, in printing the bonding between dye/pigment and fiber is analogous to dyeing, but different techniques and machinery are used. Non-limiting examples of textile printing are block printing, roller printing, screen printing, and heat transfer printing.

The term **"screen printing"** describes the process of pressing ink through a mesh screen to create a printed pattern or design.

According to one aspect, printing ink on a substrate may be performed by providing an ink, contacting the ink with the substrate, and fixing the ink on the substrate.

The term **"contacting"** as used herein refers to the step of bringing the substrate into contact with the herein described dye, the pigment, or the ink.

According to a specific aspect, contacting an ink with a substrate may be performed by any known suitable method e.g., by screen printing, roller printing, block printing, heat transfer printing, or 3D-printing.

According to one aspect, the herein described substrate is a textile material.

The term **"substrate"** as used herein refers to any material which can be a target of color changing or printing. Thereby, the substrate may be a textile material but also a raw material which is converted into a textile material such yarns or threads.

The term **"textile material"** as used herein refers to a material which is made by creating an interlocking bundle of yarns or threads, which are produced by spinning raw fibers into long and twisted lengths. The raw fibers may be of natural or synthetic sources. Textile materials are formed by weaving, knitting, crocheting, knotting, tatting, felting, bonding, or braiding these yarns together. The terms "textile material", "textile", and "fabric" may be used interchangeably herein.

The textile material can be, but is not limited to natural textile material, synthetic textile material, and combinations thereof.

The natural textile material can be, but is not limited to cotton, silk, wool, abacá, coir, linen, hemp, wood, cashmere, and mohair.

The synthetic textile material can be, but is not limited to polyester, rayon, acrylic, polycarbonate, polyethylene, polyamide, elastane, acetate, lyocell, and modal.

According to one aspect, fixing is performed by a heat treatment of the substrate.

According to specific aspect, fixing is performed by heat treatment of the substrate at a temperature in the range of 60°C to 121°C. Specifically, at a temperature in the range of 60°C to 65°C, 65°C to 70°C, 70°C to 75°C, 75°C to 80°C, 80°C to 85°C, 85°C to 90°C, 90°C to 95°C, 95°C to 100°C, 100°C to 105°C, 105°C to 110°C, 110°Cto 115°C, 115°C to 120°C, 100°C to 121°C, 115°C to 121°C, or at a temperature of 121°C. Specifically, fixing is performed by heat treatment for at least 20 minutes up to several hours. Specifically, for 20, 25, 30, 35, 40, 45, 50, 55, 60 minutes of even longer. Specifically, for 20 to 25, 20 to 30, 20 to 40, 20 to 50, or 20 to 60 minutes.

According to a specific aspect, fixing by heat treatment is performed by any method used in the field for heating a substrate. For example, the substrate may be heated in an oven or dryer. Another example of heat treatment for fixing is ironing.

According to one aspect, described herein is an ink comprising a biomass of a pigment producing microorganism, and a binder.

As described herein, a biomass may comprise a pigment and said biomass may be used for producing an ink and for changing the color of a substrate. Alternatively, such a pigment may also be isolated from the biomass and be used as an isolated pigment for e.g., producing an ink and changing the color of a substrate.

According to one aspect, a method is described herein for producing a dye, or a pigment, wherein said method comprises the steps of: providing an inoculum of the isolated bacterium deposited under the number CBS 150888 at the Westerdijk Fungal Biodiversity Institute on December 19, 2023; inoculating a cultivation medium with the inoculum; cultivating the inoculated cultivation medium; harvesting the biomass; and extracting the dye, or pigment from the harvested material.

The term **"inoculum"** as used herein refers to a population of the microorganism described herein that is introduced in a cultivation medium or any suitable medium for growing the microorganism.

According to a specific aspect, the inoculum may be in solid or liquid form. The inoculum may be a fresh culture or a taken from a frozen culture collection such as in the form of a cryo-culture. The inoculum may comprise a solid medium comprising a culture of the microorganism described herein e.g., the microorganism grown on an agar plate. Said solid inoculum may be pre-cultivated for one or more days prior to inoculation. Alternatively, the inoculum may comprise a liquid culture of the microorganism described herein. Said liquid inoculum may be pre-cultivated for one or more days prior to inoculation. Specifically, the concentration of the inoculum may vary depending on the form of the inoculum and on the pre-cultivating of the inoculum.

According to a specific aspect, pre-cultivating of a microorganism may be performed at similar conditions as cultivating.

The term **"inoculating a cultivation medium"** as used herein refers to the transfer of the inoculum into the cultivation medium.

According to one aspect, a method for changing the color of a substrate is described herein, wherein said method comprises the steps of: contacting the substrate with the dye, or the pigment described herein until the desired color is obtained; and fixing the color on the substrate.

According to one aspect, described herein is the use of a dye, or a pigment produced by the isolated bacterium deposited under the number CBS 150888 at the Westerdijk Fungal Biodiversity Institute on December 19, 2023 for changing the color of a substrate.

According to one aspect, described herein is a method of producing an ink, said method comprising mixing a dye, or a pigment produced by the isolated bacterium deposited under the number CBS 150888 at the Westerdijk Fungal Biodiversity Institute on December 19, 2023 with a binder.

According to a specific aspect, an ink described herein may comprise water.

According to one aspect, described herein is the use of an ink for changing the color of a substrate.

According to one aspect, described herein is a method of printing ink on a substrate, said method comprising the steps of: providing an ink comprising a dye, or a pigment produced by the isolated bacterium deposited under the number CBS 150888 at the Westerdijk Fungal Biodiversity Institute on December 19, 2023; contacting the ink with a substrate; and fixing the ink on the substrate.

According to one aspect, the herein described ink may further comprise water, a filler, a surfactant, a preservative, or any combination thereof.

According to a specific aspect, a textile material dyed with the dye, or the pigment described herein may have antimicrobial activity. Specifically, the growth of microorganisms on or in close proximity to the textile material is inhibited.

The examples described herein are illustrative of the present invention and are not intended to be limitations thereon. Many modifications and variations may be made to the techniques described and illustrated herein without departing from scope of the invention.

<u>EXAMPLES</u>

<u>Example 1: Production of ink</u>

**Cultivation & Growth Media**

The strain *Kocuria marina* O13 belongs to the group of pigment-producing bacterial/microbial cells. The pigment is membrane-bound, there is no water-soluble part expressed into the surrounding medium. Naturally derived, engineered as well as processed cells of *Kocuria marina* 013, partial or whole cells can be utilized as a colorant within ink and ink formulations. Pigment production is done via conventional microbial fermentation.

*Kocuria marina* 013 (*K. marina* 013) grows in mesophilic temperatures with a growth optimum at 25°C to 28°C. Cultivation is performed in complex medium e.g., Luria Bertani (LB) and Terrific Broth (TB), containing at least 10 g $l^{-1}$ peptone of casein (enzymatically digested), at least 5 g $l^{-1}$ yeast extract, at least 5 g $l^{-1}$ sodium chloride (NaCl) and, in case of TB 12.54 g $l^{-1}$ di-potassium hydrogen phosphate ($K_2HPO_4$) and 2.31 g $l^{-1}$ potassium di-hydrogen phosphate ($KH_2PO_4$). The optimum pH is within a range of 6.5 to 7.5.

Figures 2, 3, and 4 show phase-contrast microscopy of *K. marina* O13 in TB medium after 16 hours at 28°C (Fig. 2), in LB medium after 16 hours at 28°C (Fig. 3) and as concentrated harvest (Fig. 4).

Figure 5 shows the growth curve of *K. marina* O13 in LB medium during 20 hours batch cultivation at 28°C (left). In the course of the fermentation, the broth turns orange due to increased biomass which is visible in Fig. 5 (right) showing an orange-colored fermentation broth in the bioreactor.

**Separation, Purification & Drying of Microbial Biomass**

The separation of bacterial biomass and growth medium is done via centrifugation (4,800 rcf) or separation using a disk separator. The biomass in form of semi-liquid paste is washed with water or saline (0.85%) to remove salts or other components from the growth medium. The washing step is repeated twice. In a final step, the washed biomass pellet is re-suspended in a minimum of water, inactivated at 121°C for 20 minutes and frozen at a minimum of -30°C for further lyophilization processing.

Drying is performed via lyophilization or freeze-drying under vacuum for 48 to 72 hours depending on the volume. The bacterial biomass is dried to a moisture content of 5% or lower. Final grinding results in a fine powder of bacterial biomass which can be directly used as colorant in an ink composition.

**Preparation of Ink-Paste**

The fine bacterial pigment powder/bacterial biomass is mixed directly with the binder paste until a state of homogeneous distribution.

Alternatively, the fine bacterial pigment powder/ biomass is pre-suspended in a suitable amount of water and mixed until a uniform distribution of pigment and a homogeneous appearance is reached. In a second step, the binder is added, and the paste mixed again.

Suitable natural and circular binders are gum arabic (GA), carboxymethylcellulose (CMC) and guar gum (GG), chitosan, acetic acid, kaolin, methyl cellulose, sodium benzoate.

<u>Example 2: Screen Printing</u>

Textile samples chosen are **(1)** knitted, double jersey, 77% Cotton / 23% Polyester, **(2)** knitted, tricot, Carvico Vita, 78% Recycled PA / 22% Elastane, **(3)** woven, cross twill, 45% recycled Cotton / 55% organic Cotton, **(4)** woven, poplin, 100% organic Cotton GOTS, **(5)** woven, poplin, 100% Tencel.

Selected off-the-shelf ready-to-use binders are TexPro Translucent and TexPro Opaque (both Hyprprint, NL) and Togis #8160, #8161 (Togis binder; TOGIS thickener containing pure starch, Livos, DE).

Samples were screen printed with the microbial pigments from *Kocuria marina* 013 formulated as lyophilized microbial biomass. The ink comprising the pigment producing biomass from *Kocuria marina* O13 is referred to here as C19 orange. Prior to be combined with the binder, the lyophilized pigmented biomass is suspended in a small amount of water to be distributed homogeneously within the paste.

All prototypes are heat-fixated in a final step, particularly via ironing (heat-press), and subsequently, rinsed and washed with mild laundry detergent (Ecover).

Fig. 6 shows the result of the screen printing using C19 orange (ink comprising *Kocuria marina* O13). Thereby, an ink comprising 1.6 g / 8% (%w biomass /w binder) pigment was used. As binders, 20 g of binder TexPro Translucent (TPT) or Togis Binder (TGB) was used. Fig. 6 shows a light beige color formation using binder TPT and a yellow to orange color formation using binder TGB. In Fig. 6 the following is shown as A, B, C, and D:

A = n/a, control group: bacteria substrate mixed with the specific binder screenprinted onto textile, no fixation method was used

B = heat press
C = heat press + rinsing with water (after 24h)
D = heat press + washing with mild laundry detergent Ecover (after 24h).

Example 3: Screen Printing

Textile samples chosen are **(1)** knitted, double jersey, 77% Cotton / 23% Polyester, **(2)** knitted, tricot, Carvico Vita, 78% Recycled polyamide (PA) / 22% Elastane, **(3)** woven, cross twill, 45% recycled Cotton / 55% organic Cotton, **(4)** woven, poplin, 100% organic Cotton GOTS, **(5)** woven, poplin, 100% Tencel.

Selected off-the-shelf ready-to-use binders are TexPro Translucent and TexPro Opaque (both Hyprprint, NL) and Togis #8160 (Livos, DE).

Production of biomass from *Chromobacterium vaccinii* is performed similarly as described in example 1.

Samples were screen printed with the microbial pigments from *Chromobacterium vaccinii* formulated as lyophilized microbial biomass. The ink comprising the pigment producing biomass from *Chromobacterium vaccinii* is referred to here as C18 purple. Prior to be combined with the binder, the lyophilized pigmented biomass is suspended in a small amount of water to be distributed homogeneously within the paste.

All prototypes are heat-fixated in a final step, particularly via ironing (heat-press), and subsequently, rinsed and washed with mild laundry detergent (Ecover).

Fig. 7 shows the result of the screen printing using C18 purple (ink comprising *Chromobacterium vaccinii*). Thereby, an ink comprising 0.8 g / 4% (%w biomass /w binder) pigment was used. As binder, 20 g of binder TPT was used. As textile samples 77% cotton/ 23% polyester and 78% recycled PA/22% elastane was used. Fig. 7 shows a blue and not fully covering color formation using the textile sample77% cotton/ 23% polyester. Using the textile sample 78% recycled PA/22% elastane a fully covering blue color formation is shown. In Fig. 7 the following is shown as A, B, C, and D:

A = n/a, control group: bacteria substrate mixed with the specific binder screenprinted onto textile, no fixation method was used
B = heat press
C = heat press + rinsing with water (after 24h)
D = heat press + washing with mild laundry detergent Ecover (after 24h).

Example 4: Biomass and pigment production from *Kocuria marina 013*

**Production of biomass from *Kocuria marina 013***

Fermentation of *Kocuria marina* O13 in shake flask using two different growing media:

- 1 L of standard LB media (10 g peptone, 5 g yeast extract, 5 g NaCl, pH = 7=)
- 1 L 8.25 g glucose monohydrate, 10 g yeast extract, 5 g peptone, 4 g NaCl, pH = 7.9

The fermentation was performed at similar conditions as described in Example 1.

**Pigment extraction from *Kocuria marina 013***

1) The pellet was transferred to a big centrifuge bottle and put into the -20C freezer overnight.
2) To the frozen pellet methanol was added (combined mass was around 170g)
3) This mixture was then centrifugated at 5000 rpm for 10 minutes
4) The colored liquid was decanted, and step 2-3 was repeated 3 additional times.
5) The combined fractions were evaporated under reduced pressure.
6) After evaporation the orange-colored oil like substance was dissolved in minimum amounts of methanol and filtered.
7) The filtered liquid was used for UV-Vis measurements.

The extracted pigment is shown in Figure 8 which shows an orange-colored liquid in a bottle.

The UV-Vis spectrum of the extracted pigment is shown in Figure 9.

Purification of the extracted pigment to separate the different compounds present in the extract was performed by column chromatography with silica as stationary phase and methanol:dichloromethane as moving phase, with stepped

gradient elution. The extract was separatable into three parts: red part, orange part and yellow part.

The different fractions retained the trident shape in UV-Vis. Therefore, this trident was not due to mixing different colored materials, but further serves as an indicator that the colored molecules in *Kocuria marina* O13 are mixtures of different carotenoids.

Example 5: Analysis of pigments C19 and C18

Pigment C19 is the pigment extracted from *Kocuria marina* 013. The extract is used for dyeing and is a mixture of at least 3 compounds as described in example 4.

Pigment C18 is the pigment extracted from *Chromobacterium vaccinii.* The extract is used for dyeing and is a mixture of mainly violacein and deoxy violacein but a further 3 colored compounds were observed in lower quantities.

The color of the textile samples was measured using the Minolta Chrome Meter CR-200b.

L*a*b values: in the L*a*b color space developed by CIE (Commission internationale de l'eclairage) the different values determine the lightness, redness, and blueness of the measured colors.

L*c*h values: derived from L*a*b color space to make the calculated ΔE values more representative of the human eye. The values correspond to lightness, chroma, and hue.

ΔE: color difference, can be calculated with several equations such as the original CIE1972 or later iterations. The values gained from different equations are not comparable with each other. Today the standard is the ΔE2000 equation, but the textile industry also uses the $\Delta E_{CMC}$ equation as well. Contrary to the original CIE1972 equation (or $\Delta E_{ab}$), these newer iterations do not use the L*a*b values but instead the L*c*h values as these are much more representative of what the human eye can perceive. The CMC equation also has two parameters: lightness (l) and chroma (c). Commonly used values are 2:1 as the human eye is much more sensitive to chroma differences than to lightness differences and this setup doubles the tolerance of variation for lightness than that for chroma.

$$\Delta E_{ab} = \sqrt{(L_2^* - L_1^*)^2 + (a_2^* - a_1^*)^2 + (b_2^* - b_1^*)^2}$$

$$\Delta E_{2000} = \sqrt{\left(\frac{\Delta L'}{k_L S_L}\right)^2 + \left(\frac{\Delta C'}{k_C S_C}\right)^2 + \left(\frac{\Delta H'}{k_H S_H}\right)^2 + R_T \frac{\Delta C'}{k_C S_C}\frac{\Delta H'}{k_H S_H}}$$

$$\Delta E_{CMC} = \sqrt{\left(\frac{L_2^* - L_1^*}{l \times S_L}\right)^2 + \left(\frac{C_2^* - C_1^*}{c \times S_C}\right)^2 + \left(\frac{\Delta H_{ab}^*}{S_H}\right)^2}$$

Using the measurement and calculations, the observed colors during the printing and dyeing with O13 and C18 dyes are digitalized.

The Table below is about the printing results. Here O13 and C18 or violacein was used in printing trials.

| name | L | a | b | ΔE2000 | ΔECMC2:1 |
|---|---|---|---|---|---|
| 013 textile | 93.73 | 0.13 | 8.17 | | |
| 013 Blank | 91.27 | 2.07 | 17.77 | 6.57 | 6.43 |
| 013 Abrasion | 90.43 | 2.00 | 17.70 | 6.65 | 6.43 |
| 013 75% EtOH | 93.03 | 0.20 | 10.70 | 1.83 | 2.06 |
| 013 Transfer | 90.93 | 2.53 | 19.37 | 7.52 | 7.22 |
| 013 textile (washed) | 94.00 | 0.07 | 8.10 | | |
| 013 Blank (washed) | 90.83 | 2.80 | 18.57 | 7.39 | 7.06 |
| 013 Abrasion | 90.60 | 2.10 | 16.47 | 6.19 | 5.99 |
| 013 75% EtOH | 91.67 | 1.83 | 15.40 | 5.37 | 5.36 |
| 013 Transfer | 91.10 | 2.43 | 17.87 | 6.89 | 6.66 |
| C18 textile | 93.03 | 0.90 | 5.63 | | |
| C18 Blank | 63.50 | 5.80 | -12.20 | 26.09 | 23.88 |
| C18 Abrasion | 69.43 | 3.23 | -5.17 | 19.13 | 17.26 |

(continued)

| name | L | a | b | ΔE2000 | ΔECMC2:1 |
|---|---|---|---|---|---|
| C18 75% EtOH | 71.97 | 3.30 | -5.97 | 17.91 | 17.75 |
| C18 Transfer | 64.13 | 5.23 | -11.57 | 25.35 | 23.64 |
| C18 textile (washed) | 92.83 | 0.83 | 5.73 | | |
| C18 Blank (washed) | 70.13 | 3.90 | -8.37 | 20.14 | 20.79 |
| C18 Abrasion | 75.23 | 3.23 | -7.30 | 16.71 | 19.04 |
| C18 75% EtOH | 71.10 | 2.47 | -4.10 | 17.42 | 15.51 |
| C18 Transfer | 68.17 | 3.87 | -8.53 | 21.38 | 21.34 |

REFERENCES

Sharma, Gaurav; Wu, Wencheng; Dalal, Edul N. (2005). "The CIEDE2000 color-difference formula: Implementation notes, supplementary test data, and mathematical observations" (PDF). Color Research & Application. Wiley Interscience. 30 (1): 21-30. doi:10.1002/col.20070

CIE1976 formula: https://doi.ora/10.1002/i.1520-6378.1977.tb00104.x (dated December 21, 2023)

CMC:l:c formula: http://www.brucelindbloom.com/index.html?Eqn DeltaE CMC.html (dated December 21, 2023)

## Claims

1. A method of producing an ink, said method comprising the steps of:

   i. cultivating a pigment producing microorganism in a cultivation medium for producing biomass of said microorganism comprising the pigment;
   ii. separating the produced biomass from the cultivation medium;
   iii. inactivating the microorganism of the biomass;
   iv. drying the biomass;
   v. mixing the dried powder with a binder;

   wherein the pigment is membrane-bound.

2. The method of claim 1, wherein the pigment producing microorganism is an isolated bacterium belonging to the species *Kocuria marina* deposited under the number CBS 150888 at the Westerdijk Fungal Biodiversity Institute on December 19, 2023.

3. The method of claim 1 or 2, wherein cultivating is performed at least until a color of a pigment is detected in the biomass.

4. The method of any one of claims 1 to 3, wherein the method further comprises producing a powder from the dried biomass before mixing the dried biomass with the binder.

5. The method of any one of claims 1 to 4, wherein separating is performed by one or more of filtration, centrifugation, or disk separation.

6. The method of any one of claims 1 to 5, wherein the binder comprises starch, gum arabic (GA), carboxymethylcellulose (CMC), guar gum (GG), chitosan, acetic acid, kaolin, methyl cellulose, sodium benzoate, or a combination thereof.

7. An ink produced according to the method of any one of claims 1 to 6.

8. Use of an ink of claim 7 for changing the color of a substrate.

9. A method of printing ink on a substrate, said method comprising the steps of:

i. providing an ink of claim 7;
ii. contacting the ink with the substrate; and
iii. fixing the ink on the substrate, preferably by heat treatment of the substrate at a temperature in the range of 60°C to 121°C.

**10.** The use of claim 8 or the method of claim 9, wherein the substrate is a textile material.

**11.** An ink comprising a biomass of a pigment producing microorganism, and a binder, wherein the pigment is membrane-bound.

**12.** The ink of claim 11, wherein said microorganism is a bacterium, preferably a bacterium belonging to the phylum *Actinomycetota,* more preferably to the class *Actinomycetia,* even more preferably the bacterium is Kocuria sp., preferably *Kocuria marina.*

**13.** The ink of claim 11 or 12, wherein the pigment producing microorganism is an isolated bacterium belonging to the species *Kocuria marina* deposited under the number CBS 150888 at the Westerdijk Fungal Biodiversity Institute on December 19, 2023.

**14.** An isolated bacterium belonging to the species *Kocuria marina* deposited under the number CBS 150888 at the Westerdijk Fungal Biodiversity Institute on December 19, 2023.

**Patentansprüche**

**1.** Verfahren zur Herstellung einer Tinte, wobei das Verfahren die Schritte umfasst:

i. Kultivieren eines ein Pigment produzierenden Mikroorganismus in einem Kulturmedium, um Biomasse des Mikroorganismus zu produzieren, welche das Pigment umfasst;
ii. Abtrennen der produzierten Biomasse von dem Kulturmedium;
iii. Inaktivieren des Mikroorganismus der Biomasse;
iv. Trocknen der Biomasse;
v. Mischen des getrockneten Pulvers mit einem Bindemittel, wobei das Pigment membrangebunden ist.

**2.** Verfahren nach Anspruch 1, wobei der ein Pigment produzierende Mikroorganismus ein isoliertes Bakterium ist, das zu der Art *Kocuria marina* gehört, die unter der Kennung CBS 150888 am 19. Dezember 2023 bei dem Westerdijk Fungal Biodiversity Institute hinterlegt wurde.

**3.** Verfahren nach Anspruch 1 oder 2, wobei das Kultivieren zumindest solange durchgeführt wird, bis eine Farbe eines Pigments in der Biomasse detektiert wird.

**4.** Verfahren nach einem der Ansprüche 1 bis 3, wobei das Verfahren ferner das Produzieren eines Pulvers aus der getrockneten Biomasse vor dem Mischen der getrockneten Biomasse mit dem Bindemittel umfasst.

**5.** Verfahren nach einem der Ansprüche 1 bis 4, wobei das Abtrennen durch eines oder mehrere aus Filtration, Zentrifugation oder Scheibentrennung durchgeführt wird.

**6.** Verfahren nach einem der Ansprüche 1 bis 5, wobei das Bindemittel Stärke, Gummi arabicum (GA), Carboxymethylcellulose (CMC), Guargummi (GG), Chitosan, Essigsäure, Kaolin, Methylcellulose, Natriumbenzoat oder eine Kombination davon umfasst.

**7.** Tinte, produziert gemäß dem Verfahren nach einem der Ansprüche 1 bis 6.

**8.** Verwendung einer Tinte nach Anspruch 7 zur Veränderung der Farbe eines Substrats.

**9.** Verfahren zum Drucken einer Tinte auf ein Substrat, wobei das Verfahren die Schritte umfasst:

i. Bereitstellen einer Tinte nach Anspruch 7;
ii. Inkontaktbringen der Tinte mit dem Substrat; und

iii. Fixieren der Tinte auf dem Substrat, vorzugsweise durch Wärmebehandlung des Substrats bei einer Temperatur in dem Bereich von 60 °C bis 121 °C.

**10.** Verwendung nach Anspruch 8 oder Verfahren nach Anspruch 9, wobei das Substrat ein textiles Material ist.

**11.** Tinte, umfassend eine Biomasse eines ein Pigment produzierenden Mikroorganismus und ein Bindemittel, wobei das Pigment membrangebunden ist.

**12.** Tinte nach Anspruch 11, wobei der Mikroorganismus ein Bakterium ist, vorzugsweise ein Bakterium, das zu dem Stamm *Actinomycetota,* noch bevorzugter zu der Klasse *Actinomycetia* gehört, wobei das Bakterium sogar noch bevorzugter Kocuria sp. ist, vorzugsweise *Kocuria marina.*

**13.** Tinte nach Anspruch 11 oder 12, wobei der das Pigment produzierende Mikroorganismus ein isoliertes Bakterium ist, das zu der Art *Kocuria marina* gehört, die unter der Kennung CBS 150888 am 19. Dezember 2023 bei dem Westerdijk Fungal Biodiversity Institute hinterlegt wurde.

**14.** Isoliertes Bakterium, das zu der Art *Kocuria marina* gehört, die unter der Kennung CBS 150888 am 19. Dezember 2023 bei dem Westerdijk Fungal Biodiversity Institute hinterlegt wurde.

## Revendications

**1.** Procédé de production d’une encre, ledit procédé comprenant les étapes de :

i. culture d’un microorganisme producteur de pigment dans un milieu de culture permettant de produire de la biomasse dudit microorganisme comprenant le pigment ;
ii. séparation de la biomasse produite du milieu de culture ;
iii. inactivation du microorganisme de la biomasse ;
iv. séchage de la biomasse ;
v. mélange de la poudre sèche avec un liant ;

dans lequel le liant est lié à une membrane.

**2.** Procédé de la revendication 1, dans lequel le microorganisme producteur de pigment est une bactérie isolée appartenant à l’espèce *Kocuria marina* déposée sous le numéro CBS 150888 auprès du Westerdijk Fungal Biodiversity Institute le 19 décembre 2023.

**3.** Procédé de la revendication 1 ou 2, dans lequel la culture est effectuée au moins jusqu’à ce qu’une couleur d’un pigment soit détectée dans la biomasse.

**4.** Procédé selon l’une quelconque des revendications 1 à 3, dans lequel le procédé comprend en outre la production d’une poudre à partir de la biomasse séchée avant le mélange de la biomasse séchée au liant.

**5.** Procédé de l’une quelconque des revendications 1 à 4, dans lequel la séparation est effectuée par une ou plusieurs opérations parmi une filtration, une centrifugation ou une séparation par disques.

**6.** Procédé de l’une quelconque des revendications 1 à 5, dans lequel le liant comprend de l’amidon, de la gomme arabique (GA), de la carboxyméthylcellulose (CMC), de la gomme de guar (GG), du chitosane, de l’acide acétique, du kaolin, de la méthylcellulose, du benzoate de sodium, ou une combinaison de ceux-ci.

**7.** Encre produite selon le procédé de l’une quelconque des revendications 1 à 6.

**8.** Utilisation d’une encre de la revendication 7 pour le changement de couleur d’un substrat.

**9.** Procédé d’impression d’une encre sur un substrat, ledit procédé comprenant les étapes de :

i. fourniture d’une encre de la revendication 7 ;
ii. mise en contact de l’encre avec le substrat ; et

iii. fixation de l'encre sur le substrat, de préférence par traitement thermique du substrat à une température comprise dans la plage de 60°C à 121°C.

**10.** Utilisation de la revendication 8 ou la revendication 9, dans laquelle le substrat est un matériau textile.

**11.** Encre comprenant une biomasse d'un microorganisme producteur de pigment, et un liant, dans laquelle le pigment est lié à une membrane.

**12.** Encre de la revendication 11, dans laquelle ledit microorganisme est une bactérie, de préférence une bactérie appartenant au phylum *Actinomycetota,* plus préférablement à la classe *Actinomycetia,* encore plus préférablement la bactérie est Kocuria sp., de préférence *Kocuria marina.*

**13.** Encre selon la revendication 11 ou 12, dans laquelle le microorganisme producteur de pigment est une bactérie isolée appartenant à l'espèce *Kocuria marina* déposée sous le numéro CBS 150888 auprès du Westerdijk Fungal Biodiversity Institute le 19 décembre 2023.

**14.** Bactérie isolée appartenant à l'espèce *Kocuria marina* déposée sous le numéro CBS 150888 auprès du Westerdijk Fungal Biodiversity Institute le 19 décembre 2023.

Fig.1

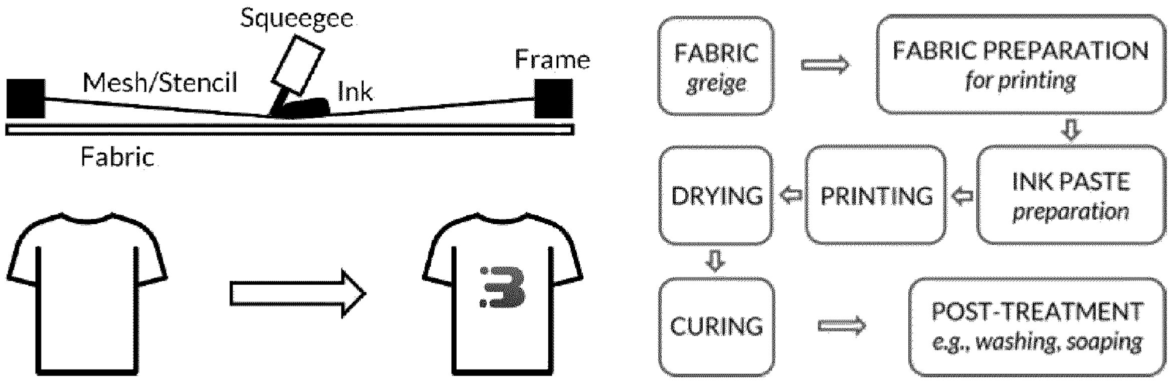
Squeegee
Frame
Mesh/Stencil
Ink
Fabric
FABRIC
greige
FABRIC PREPARATION
for printing
INK PASTE
preparation
PRINTING
DRYING
CURING
POST-TREATMENT
e.g., washing, soaping

Fig. 2

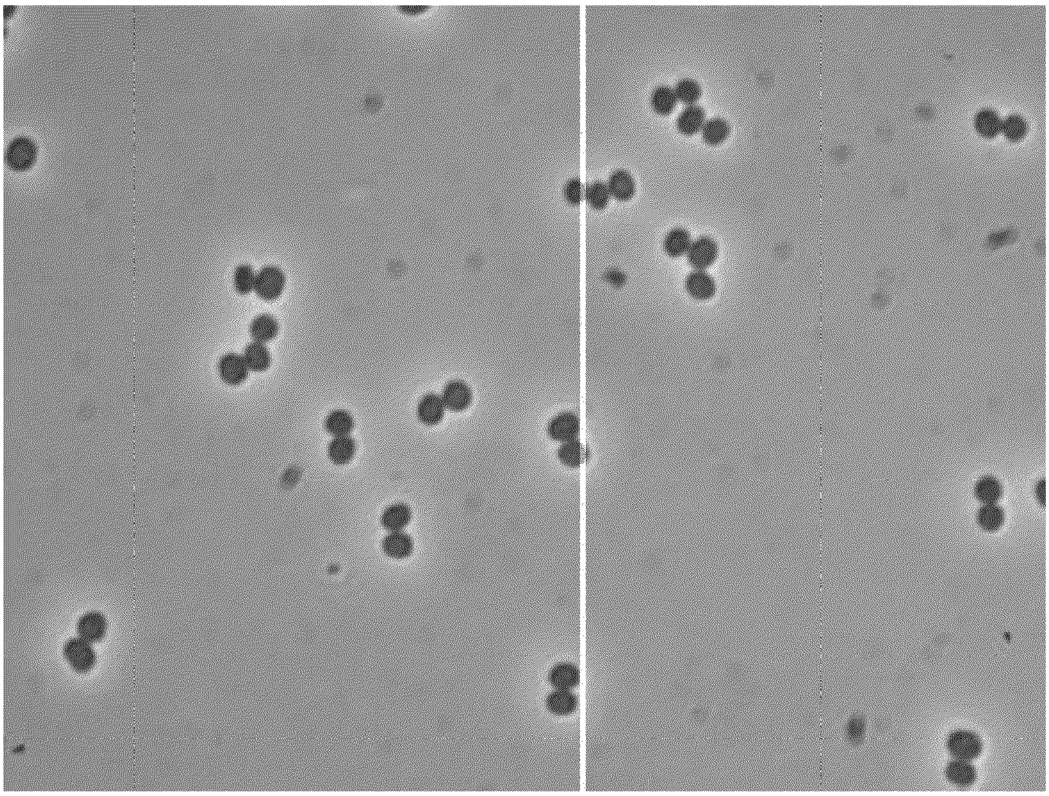

Fig.3

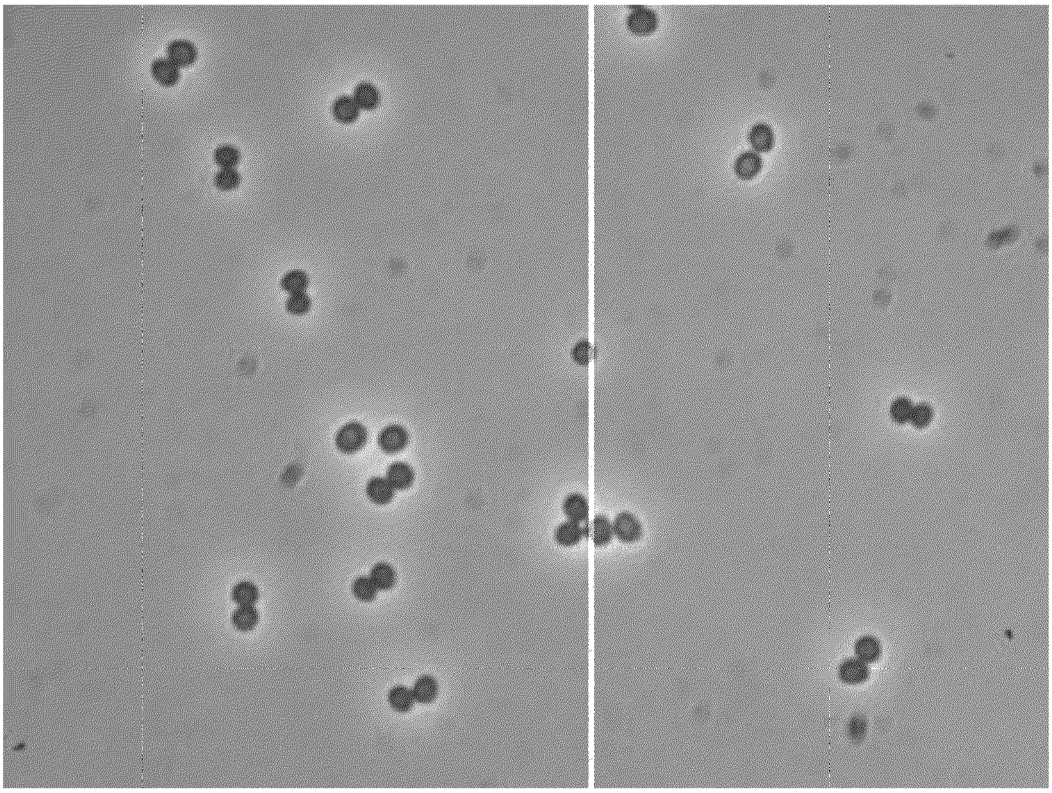

Fig. 4

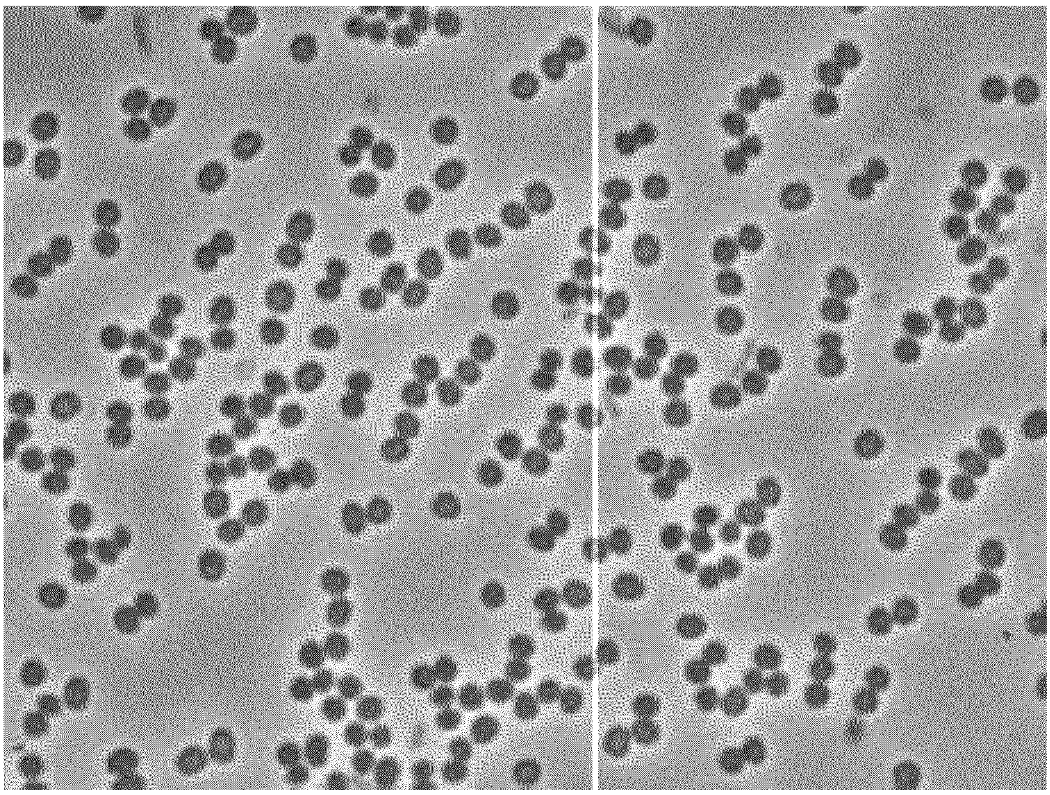

Fig. 5

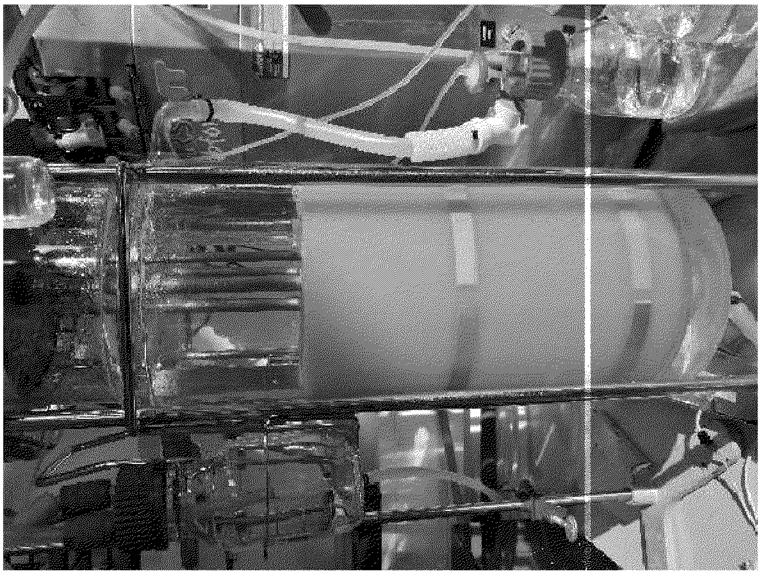

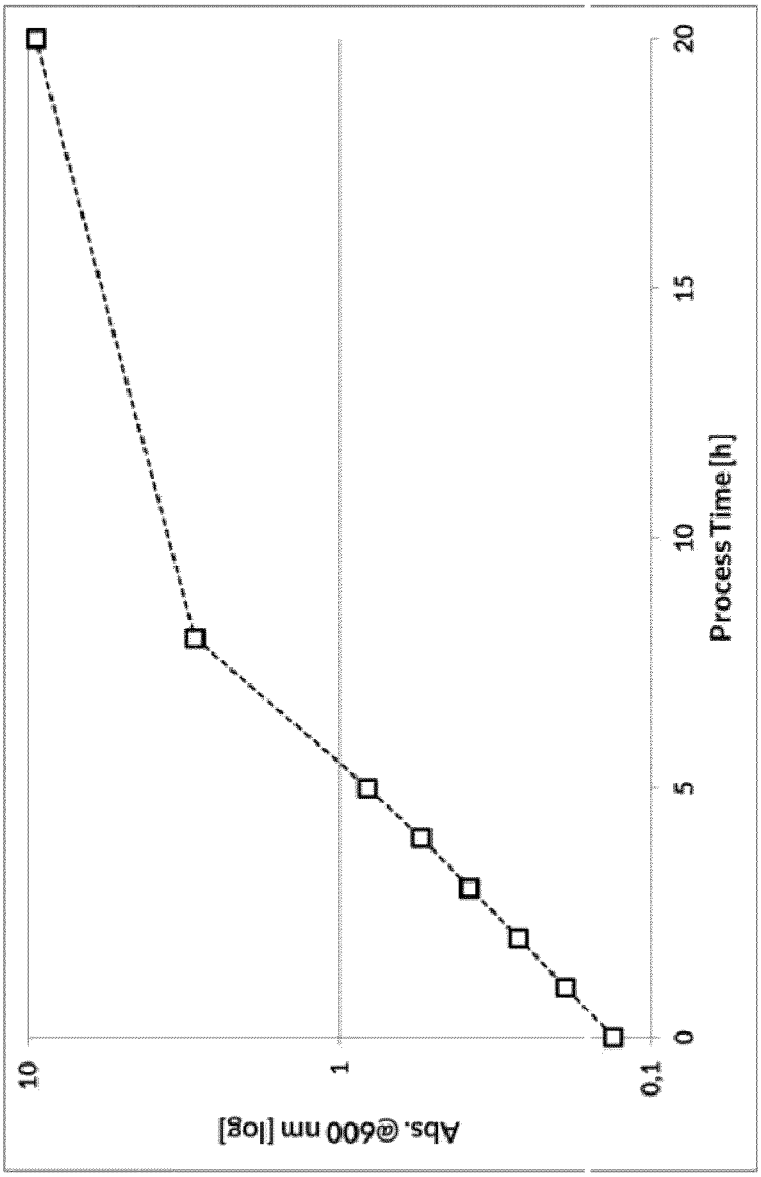

Fig. 6

1,6 g / 8% pigment – 20 g binder

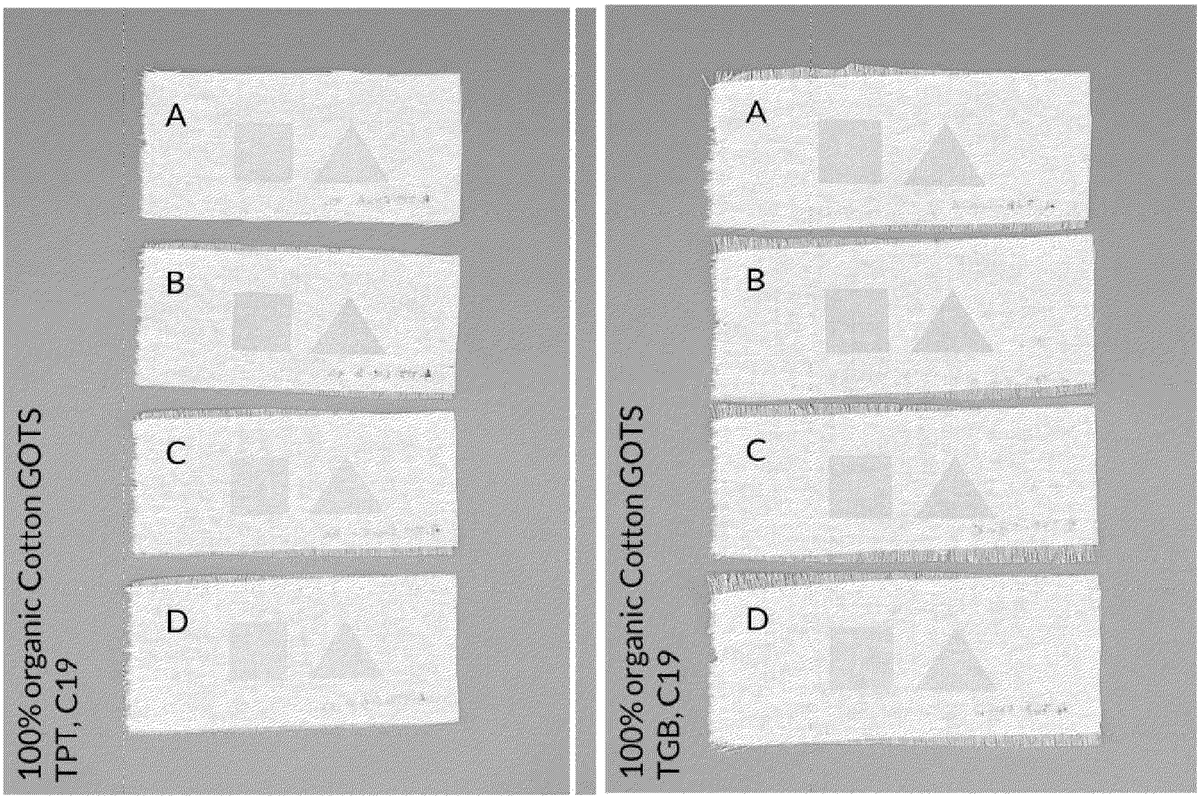

Fig. 7

0.8 g /4% pigment - 20 g binder

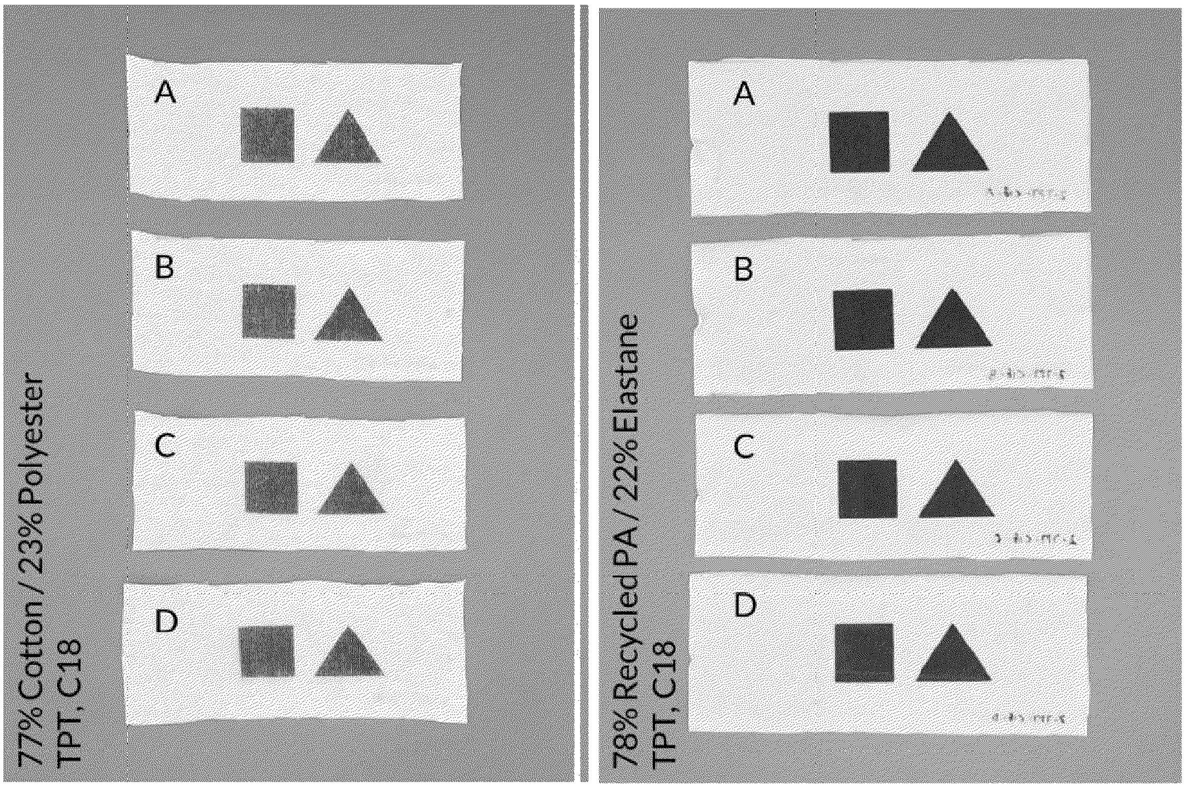


Fig. 8

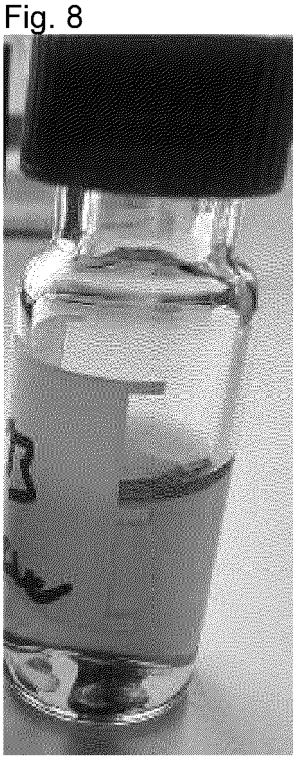

Fig. 9

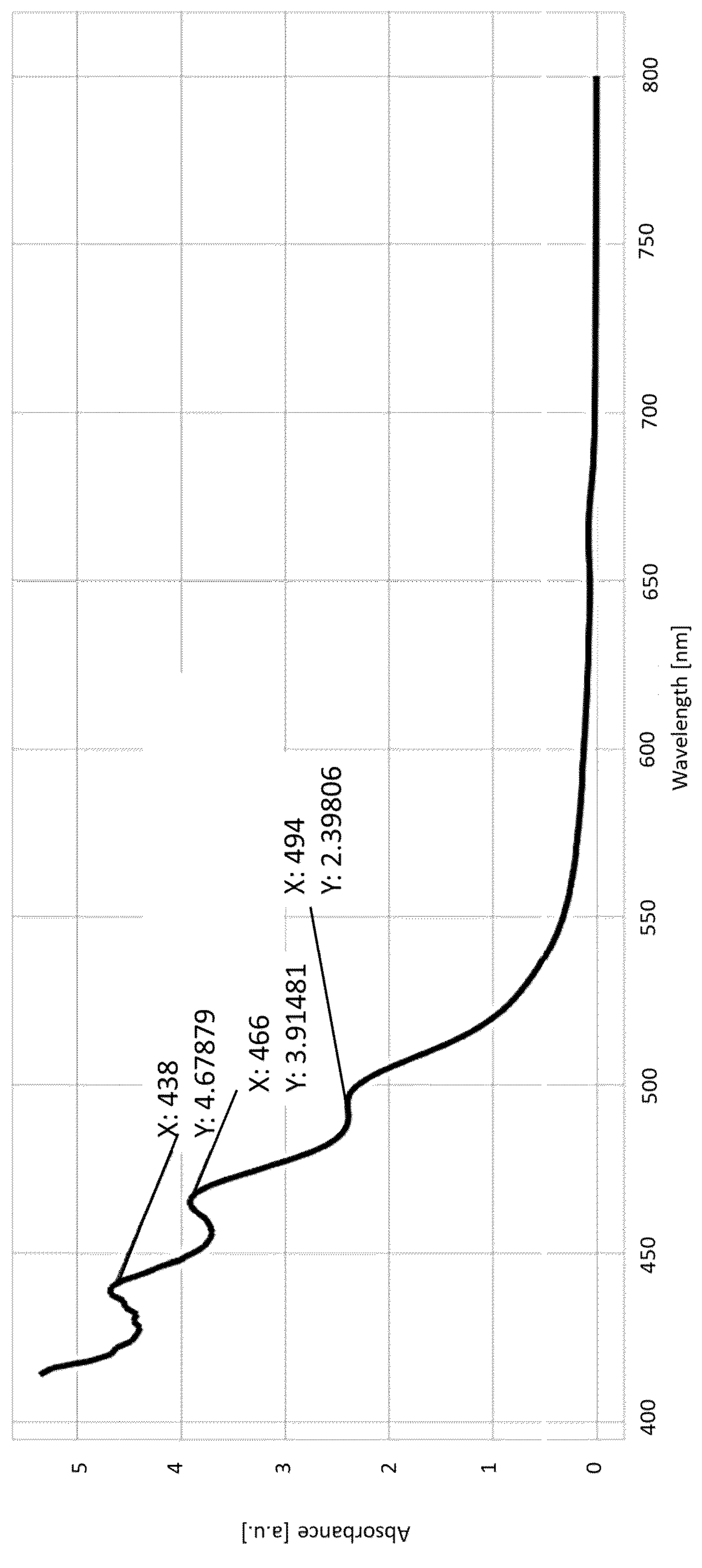
X: 438
Y: 4.67879
X: 466
Y: 3.91481
X: 494
Y: 2.39806
Absorbance [a.u.]
5
4
3
2
1
0
400
450
500
550
600
650
700
750
800
Wavelength [nm]

Fig 10

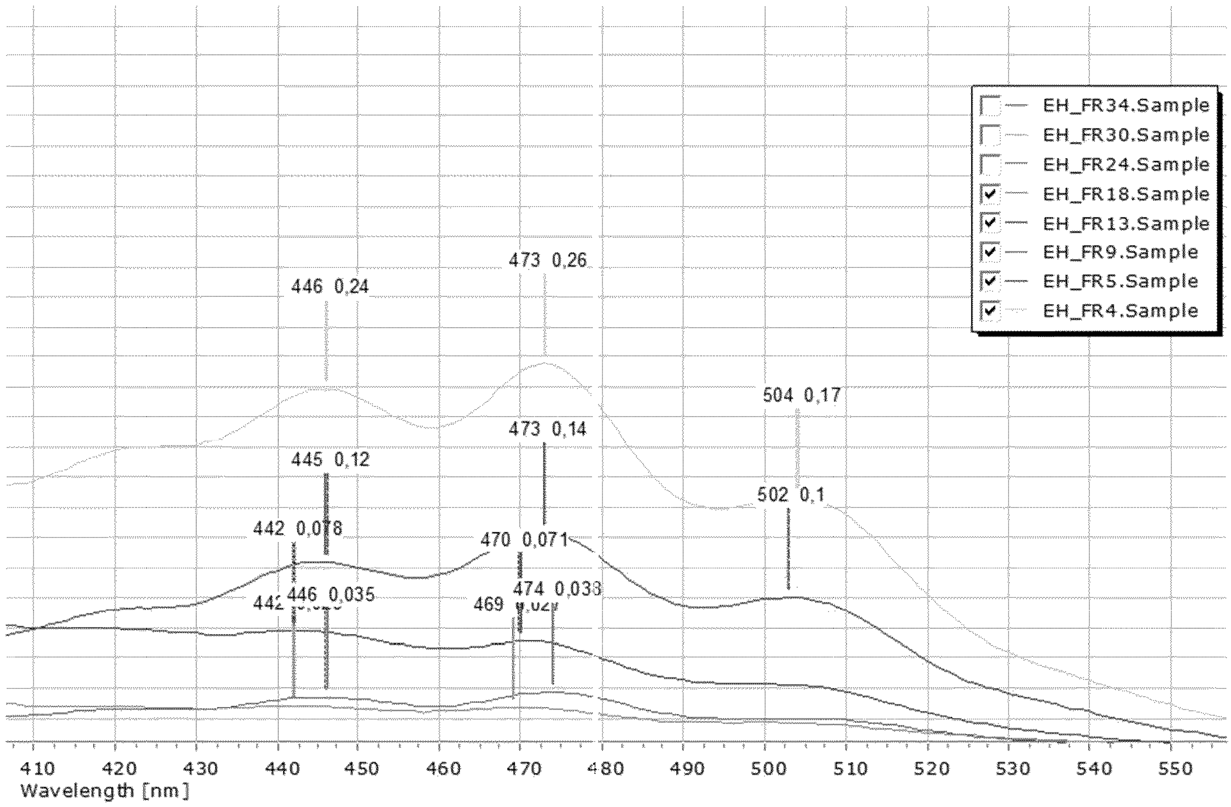
EH_FR34.Sample
EH_FR30.Sample
EH_FR24.Sample
EH_FR18.Sample
EH_FR13.Sample
EH_FR9.Sample
EH_FR5.Sample
EH_FR4.Sample
473 0,26
446 0,24
504 0,17
473 0,14
445 0,12
502 0,1
442 0,078
470 0,071
446 0,035
474 0,033
442
469
410
420
430
440
450
460
470
480
490
500
510
520
530
540
550
Wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0252002 A2
- FR 3062130 A1
- WO 2004048589 A1


### Non-patent literature cited in the description


- **SAMBROOK et al.** Molecular Cloning: A Laboratory Manual. Cold Spring Harbor Laboratory Press, 2012, vol. 1 -3
- **KREBS et al.** Lewin's Genes XI. Jones & Bartlett Learning, 2017
- **BERG et al.** Stryer Biochemie. Springer Verlag, 2018
- **ITTEN J.** *Kunst der Farbe*, 2021
- **ST. CLAIR K.** *Het geheime leven van kleuren*, 2017
- **BOOTH A.** *The Wild Dyer*, 2017
- **WADA S.** *Dictionary Of Color Combinations*, 2011, vol. 1
- **PERRYMAN L.** *The Colour Bible*, 2021
- **HANS G. VÖLZ**. Industrial Color Testing: Fundamentals and Techniques.. Wiley-VCH, 2001
- The CIEDE2000 color-difference formula: Implementation notes, supplementary test data, and mathematical observations. **SHARMA, GAURAV** ; **WU, WENCHENG** ; **DALAL, EDUL N.** Color Research & Application.. Wiley Interscience., 2005, vol. 30, 21-30